# EUROPÄISCHE PATENTSCHRIFT

(11) **EP 1 349 645 B1**
(45) Veröffentlichungstag und Bekanntmachung des Hinweises auf die Patenterteilung: **31.08.2005**
(21) Anmeldenummer: 01998392.3
(22) Anmeldetag: 17.11.2001
(51) Int. Cl.: B01D 71/02, A61M 1/14, A61M 1/16

(54) **WERKSTOFF ZUR BEGRENZTEN SELEKTIVEN ADSORPTION UND/ODER ABSORPTION VON KÖRPEREIGENEN SUBSTANZEN IM BLUT**
SUBSTANCE FOR LIMITED SELECTIVE ADSORPTION AND/OR ABSORPTION OF ENDOGENIC SUBSTANCES IN THE BLOOD
MATIERE D'ADSORPTION ET/OU D'ABSORPTION SELECTIVE LIMITEE DE SUBSTANCES DU CORPS DANS LE SANG

(30) Priorität: 29.11.2000 DE 10059198
(43) Veröffentlichungstag der Anmeldung: 08.10.2003
(73) Patentinhaber: Intech Thüringen GmbH, 99880 Waltershausen (DE)
(72) Erfinder: SCHMAUDER, Hans-Peter, 07745 Jena (DE); SCHUNK, Werner, 99867 Gotha (DE); BRUDER, Michael, 22147 Hamburg (DE); HEIBER, Uwe, 99085 Erfurt (DE); KRAUSE, Karl-Heinz, 09123 Chemnitz (DE); MERKMANN, Gerhard, 99867 Gotha (DE)
(86) Internationale Anmeldenummer: PCT/DE2001/004334
(87) Internationale Veröffentlichungsnummer: WO 2002/043843

(56) Entgegenhaltungen:
- DD-A- 270 867
- DE-A- 1 720 087
- DE-A- 2 705 735
- DE-A- 2 722 025
- DE-A- 3 110 128

## Beschreibung

Die Erfindung betrifft einen Werkstoff zur begrenzten selektiven Adsorption und/oder Absorption von körpereigenen Substanzen im Blut, die bei Überschreitung der entsprechenden Normwerte stoffwechselschädigend sind, umfassend wenigstens:
- eine blutdurchlässige und blutverträgliche Matrix, insbesondere in Form einer Membran; sowie
- einen Träger in Form eines Molekularsiebes, der in die Matrix eingearbeitet bzw. matrixmäßig verarbeitet und mit wenigstens einer körpereigenen Substanz und ferner mit einem Antithrombotikum beladen ist, und zwar unter Bildung eines Molekularsieb/Substanz/Antithrombotikum-Adduktes, wobei die Konzentration der beladenen körpereigenen Substanz unterhalb ihres Normwertes liegt, so dass auf Grund des Konzentrationsgefälles dieser Substanz innerhalb des Systems von Blut und Matrix die Adsorption und/oder Absorption der stoffwechselschädigenden Substanz bis zur Wiedereinstellung des Normwertes erfolgt, wobei ferner gleichzeitig das Antithrombotikum desorptiv freigegeben wird.

Körpereigene Substanzen im Blut, beispielsweise Harnstoff, Mineralstoffe, Spurenelemente, Blutfette (Cholesterin), unterliegen bestimmten Toleranzgrenzen (Normwerte), die nicht längere Zeit überschritten werden dürfen, da ansonsten Krankheitssymptome auftreten. Stoffwechselschädigende Störungen erfassen dabei insbesondere das Herz-Kreislauf-System, die Gefäße, Leber und die Nieren.

Zur Reduktion der bereits eingetretenen Erkrankungen wird nun die selektive Adsorption und/oder Absorption von körpereigenen Substanzen zu einer wichtigen Therapiemaßnahme. Als besonderer Anwendungsfall soll hier die Dialyse genannt sein, die hier auch den nächstliegenden Stand der Technik beinhaltet, wobei beispielsweise auf die Offenlegungsschrift DE 197 15 504 A1 verwiesen wird.

In der Patentschrift DD 270 867 A1 wird ein Mittel zur Entfernung toxischer Substanzen aus dem Blut vorgestellt. Dabei wird ein Molekularsieb bei einem pH-Wert von 7,4 ± 0,4 mit einer dem Blut entsprechenden Kationenzusammensetzung und Kationenkonzentration beladen.

Im Rahmen einer Weiterentwicklung besteht nun die Aufgabe darin, einen Werkstoff bereitzustellen, der die Wirksamkeit der selektiven Adsorption und/oder Absorption verbessert, verbunden mit der Maßgabe, die proinflammatorischen (entzündungsfördernden) Faktoren im Körper zu reduzieren oder zu steuem. Darüber hinaus soll die Einhaltung des pH-Wertes von 7,5 ± 0,5 im Blut gewährleistet sein.

Zwecks Lösung dieser Aufgabe zeichnet sich nun der erfindungsgemäße Werkstoff dadurch aus, dass das Molekularsieb/Substanz/Antithrombotikum-Addukt zusätzlich Kristallwasser enthält, und zwar derart, dass in Bezug auf eine Grundmolmenge (m) an Kristallwasser von wenigstens 25 das Molekularsieb bei einem Dehydratisierungsgrad von mindestens 20% partiell dehydratisiert ist, wobei das Molekularsieb mit der reduzierten Molmenge (m') an Kristallwasser mit der körpereigenen Substanz und dem Antithrombotikum beladen ist, so dass bei Blutkontakt der Matrix eine Adsorption von Wasser unter Desorption des Antithrombotikums stattfindet, wobei der Kristallwassergehalt des Molekularsiebes zunimmt.

Das Molekularsieb ist insbesondere ein Metall-Aluminium-Silikat der folgenden Formel:

Meₙ [(AlO₂)_{X} · (SiO₂)_{Y}] · m(m')H₂O

Das Molekularsieb ist in Bezug auf die Grundmolmenge (m) an Kristallwasser bei einem Dehydratisierungsgrad von vorzugsweise 30 % bis 70 % partiell dehydratisiert.

Im Folgenden werden zwei besondere Anwendungsfälle (A, B) des neuen Werkstoffes vorgestellt:
A) Der Werkstoff hat einen im Wesentlichen hydrophoben Charakter, wobei die Grundmolmenge (m) an Kristallwasser insbesondere im Bereich von 25 bis 50 liegt. In diesem Zusammenhang ist vor allem das Natrium-Aluminium-Silikat der folgenden Formel zu nennen:

   Na₁₂ [(AlO₂)₁₂ · (SiO₂)₁₂] · 27 H₂O

   Nach der partiellen Dehydratisierung ist das Molekularsieb mit der reduzierten Molmenge (m'; z.B. m' = 15) mit der körpereigenen Substanz und dem Antithrombotikum beladen, wobei dann das Addukt in Wechselwirkung mit dem Blut seine Wirkung entfaltet.
B) Der Werkstoff hat einen hydrophilen Charakter, wobei die Grundmolmenge (m) an Kristallwasser wenigstens 100, insbesondere wenigstens 200, beträgt. Hier ist vorzugsweise das Natrium-Aluminium-Silikat der folgenden Formel zu nennen:

   Na₈₆ [(AlO₂)₈₆ · (SiO₂)₁₀₆] · 276 H₂O

   Wiederum nach der partiellen Dehydratisierung ist das Molekularsieb mit der reduzierten Molmenge (m'; z.B. m' = 150) mit der körpereigenen Substanz und dem Antithrombotikum beladen, wobei dann auch hier das Addukt in Wechselwirkung mit dem Blut seine Wirkung entfaltet.

Dadurch dass das Molekularsieb/Substanz/Antithrombotikum-Addukt zusätzlich Kristallwasser enthält, können in Verbindung mit der Molmenge (m, m') an Kristallwasser die proinflammatorischen Faktoren im Körper entsprechend gesteuert werden.

Außerdem trägt das Kristallwasser zur verbesserten Einhaltung des pH-Wertes von 7,5 ± 0,5 im Blut bei. Die Einhaltung dieses pH-Wertes ist eine wichtige Voraussetzung auf die Einflussnahme der anderen Blutbestandteile. Gegebenenfalls kann das partiell dehydratisierte Molekularsieb zusätzlich mit Mineralien beladen sein, die den pH-Wert im Blut regulieren.

Die weiteren zweckmäßigen Werkstoffparameter sind:
- Der Gesamtbeladungsgrad der körpereigenen Substanz und des Antithrombotikums ist kleiner als der Dehydratisierungsgrad des partiell dehydratisierten Molekularsiebes. Der Gesamtbeladungsgrad ist dabei mindestens 20 % des Dehydratisierungsgrades.
- Das Addukt ist innerhalb der Matrix im Wesentlichen gleichmäßig verteilt.
- Das Addukt weist einen Anteil von 2 bis 30 Gew.-%, vorzugsweise 15 bis 25 Gew.-%, auf, und zwar bezogen auf die Gesamtmasse der Matrix. Die Beladungsmenge der körpereigenen Substanz beträgt 2 bis 20 Gew.-% und die des Antithrombotikums 1 bis 10 Gew.-%, und zwar jeweils bezogen auf die Gesamtmasse des Adduktes.
- Die Matrix ist ein Polymerwerkstoff, insbesondere wiederum ein Elastomer, ein thermoplastisches Elastomer oder ein thermoplastischer Kunststoff. Wichtig in diesem Zusammenhang ist, dass diese Werkstoffe blutdurchlässig und blutverträglich sind.

Darüber hinaus besteht die Aufgabe darin, ein Verfahren zur Herstellung des erfindungsgemäßen Werkstoffes bereitzustellen.

Das Verfahren zur Herstellung des neuen Werkstoffes zeichnet sich nun durch folgende Verfahrensschritte aus:
- das Molekularsieb mit einer Grundmolmenlge (m) an Kristallwasser von wenigstens 25 wird bei 200 bis 300°C, vorzugsweise bei etwa 250°C, mehrere Stunden, vorzugsweise 2 bis 6 Stunden, lang bei einem Dehydratisierungsgrad von mindestens 20% partiell dehydratisiert;
- anschließend wird das partiell dehydratisierte Molekularsieb mit der reduzierten Molmenge (m') an Kristallwasser mit wenigstens einer körpereigenen Substanz und dem Antithrombotikum sowie gegebenenfalls mit weiteren Stoffen unter Bildung des Adduktes beladen;
- schließlich wird das Addukt in die Matrix eingearbeitet bzw. matrixmäßig verarbeitet.

Die Dehydratisierung und/oder Beladung wird/werden dabei insbesondere in Gegenwart eines inerten Gases, beispielsweise von Stickstoff, durchgeführt. Die Dehydratisierung und/oder Beladung erfolgt/erfofgen ferner vorzugsweise unter Normaldruck. Die Beladung selbst kann beispielsweise mittels einer Kugelmühle vorgenommen werden.

Die in der Beschreibung und in den Patentansprüchen erwähnten Aussagen zum Dehydratisierungsgrad und Beladungsgrad beziehen sich auf den Zustand nach Abschluss der partiellen Dehydratisierung bzw. der Beladung, d.h. ohne Stoffwechselmechanismus.

Die Erfindung wird nun anhand eines Ausführungsbeispieles unter Bezugnahme auf eine schematische Zeichnung erläutert.

Die Dialyse-Membran **1** umfasst eine Matrix **2,** in die das Molekularsieb/Substanz/Antithrombotikum-Addukt **3** eingearbeitet (eingemisch) ist, wobei das Molekularsieb partiell dehydratisiert ist. Ferner ist das Addukt innerhalb der Matrix im Wesentlichen gleichmäßig verteilt.

Die Konzentration der beladenen körpereigenen Substanz X (hier Harnstoff) liegt unterhalb ihres Normwertes. Im Blut **4** selbst liegt die Konzentration der körpereigenen Substanz X' (hier ebenfalls Harnstoff) stoffwechselschädigend über ihrem Normwert. Auf Grund des Konzentrationsgefälles des Harnstoffes innerhalb des Systems von Blut und Dialyse-Membran erfolgt nun die Adsorption der stoffwechselschädigenden Substanz X' bis zur Erreichung des sich einstellenden dynamischen Gleichgewichtes an der Grenzfläche **5** von Dialyse-Membran und Blut. Somit wird die Wiedereinstellung des Normwertes erzielt.

Gleichzeitig findet ferner eine Adsorption von Wasser als Blutbestandteil unter Desorption des Antithrombotikums Y statt. Im Zusammenhang mit der Verwendung eines partiell dehydratisierten Molekularsiebes kann über den Dehydratisierungsgrad die Abgabegeschwindigkeit des Antithrombotikums gesteuert werden. So wird beispielsweise ein Addukt **3** mit einem Dehydratisierungsgrad von 60 % begieriger Wasser aufnehmen als ein Addukt mit einem Dehydratisierungsgrad von 30 %, und zwar in Bezug auf die gleiche Beladungsmenge an Antithrombotikum.

Die Membran **1** kann beispielsweise in folgenden Formen auftreten, und zwar als:
- strukturierte Membran (porenartig, schaumartig);
- perforierte Membran;
- sorptionsblockartige Membran.

Das erfindungsgemäße Konzept, das anhand des Ausführungsbeispieles nur eine einzige körpereigene Substanz X innerhalb des Adduktes **3** aufweist, kann auch angewendet werden, wenn das partiell dehydratisierte Molekularsieb mit zwei oder mehreren körpereigenen Substanzen beladen ist, verbunden mit der selektiven Adsorption und/oder Absorption von entsprechenden stoffwechselschädigenden körpereigenen Substanzen.

### Bezugszeichenliste

- **1**: Membran (Dialyse-Membran)
- **2**: Matrix
- **3**: Träger/Substanz/Antithrombotikum-Addukt
Molekularsieb/Substanz/Antithrombotikum-Addukt
- **4**: Blut
- **5**: Grenzfläche von Membran und Blut
- X: körpereigene Substanz als Adduktbestandteil
- X': körpereigene Substanz im Blut
- Y: Antithrombotikum

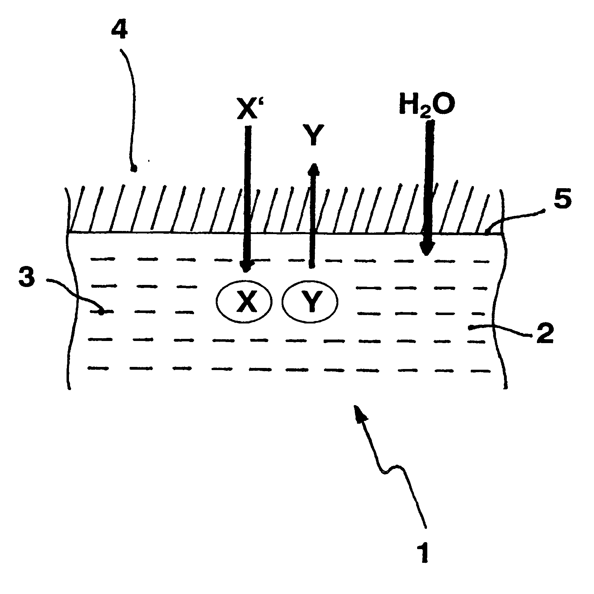

## Patentansprüche

1. Werkstoff zur begrenzten selektiven Adsorption und/oder Absorption von körpereigenen Substanzen im Blut, die bei Überschreitung der entsprechenden Normwerte stoffwechselschädigend sind, umfassend wenigstens:
- eine blutdurchlässige und blutverträgliche Matrix (2), insbesondere in Form einer Membran (1); sowie
- einen Träger in Form eines Molekularsiebes, der in die Matrix (2) eingearbeitet bzw. matrixmäßig verarbeitet und mit wenigstens einer körpereigenen Substanz (X) und ferner mit einem Antithrombotikum (Y) beladen ist, und zwar unter Bildung eines Molekularsieb/Substanz/Antithrombotikum-Adduktes (3), wobei die Konzentration der beladenen körpereigenen Substanz (X) unterhalb ihres Normwertes liegt, so dass auf Grund des Konzentrationsgefälles dieser Substanz innerhalb des Systems von Blut (4) und Matrix (2) die Adsorption und/oder Absorption der stoffwechselschädigenden Substanz (X') bis zur Wiedereinstellung des Normwertes erfolgt, wobei ferner gleichzeitig das Antithrombotikum (Y) desorptiv freigegeben wird;
**dadurch gekennzeichnet, dass**
- das Molekularsieb/Substanz/Antithrombotikum-Addukt (3) zusätzlich Kristallwasser enthält, und zwar derart, dass in Bezug auf eine Grundmolmenge (m) an Kristallwasser von wenigstens 25 das Molekularsieb bei einem Dehydratisierungsgrad von mindestens 20% partiell dehydratisiert ist, wobei das Molekularsieb mit der reduzierten Molmenge (m') an Kristallwasser mit der körpereigenen Substanz (X) und dem Antithrombotikum (Y) beladen ist, so dass bei Blutkontakt der Matrix (2) eine Adsorption von Wasser unter Desorption des Antithrombotikums stattfindet, wobei der Kristallwassergehalt des Molekularsiebes zunimmt.

2. Werkstoff nach Anspruch 1, **dadurch gekennzeichnet, dass** das Molekularsieb ein Metall-Aluminium-Silikat der folgenden Formel ist:
Meₙ [(AlO₂)_{X}· (SiO₂)_{Y}]· m'H₂O

3. Werkstoff nach Anspruch 2, **dadurch gekennzeichnet, dass** ein Metall der ersten oder zweiten Hauptgruppe des Periodensystems, vorzugsweise Natrium, Verwendung findet.

4. Werkstoff nach Anspruch 2 oder 3, **dadurch gekennzeichnet dass** das Molekularsieb ein Natrium-Aluminium-Silikat der folgenden Formel ist:
Na₁₂ [(AlO₂)₁₂ · (SiO₂)₁₂] · m'H₂O

5. Werkstoff nach Anspruch 2 oder 3, **dadurch gekennzeichnet, dass** das Molekularsieb ein Natrium-Aluminium-Silikat der folgenden Formel ist:
Na₈₆ [(AlO₂)₈₈ · (SiO₂)₁₀₆] · m' H₂O

6. Werkstoff nach einem der Ansprüche 1 bis 5, **dadurch gekennzeichnet, dass** das Molekularsieb eine Grundmolmenge (m) an Kristallwasser von wenigstens 100, insbesondere wenigstens 200, enthält.

7. Werkstoff nach einem der Ansprüche 1 bis 6, **dadurch gekennzeichnet, dass** das partiell dehydratisierte Molekularsieb einen Dehydratisierungsgrad von 30 % bis 70 % aufweist.

8. Werkstoff nach einem der Ansprüche 1 bis 7, **dadurch gekennzeichnet, dass** der Gesamtbeladungsgrad der körpereigenen Substanz (X) und des Antithrombotikums (Y) kleiner ist als der Dehydratisierungsgrad des partiell dehydratisierten Molekularsiebes.

9. Werkstoff nach Anspruch 8, **dadurch gekennzeichnet, dass** der Gesamtbeladungsgrad mindestens 20 % des Dehydratisierungsgrades beträgt.

10. Werkstoff nach einem der Ansprüche 1 bis 9, **dadurch gekennzeichnet, dass** das Addukt (3) innerhalb der Matrix (2) im Wesentlichen gleichmäßig verteilt ist.

11. Werkstoff nach einem der Ansprüche 1 bis 10, **dadurch gekennzeichnet, dass** das Addukt (3) einen Anteil von 2 bis 30 Gew.-%, vorzugsweise 15 bis 25 Gew.-%, aufweist, und zwar bezogen auf die Gesamtmasse der Matrix (2).

12. Werkstoff nach einem der Ansprüche 1 bis 11, **dadurch gekennzeichnet, dass** die Beladungsmenge der körpereigenen Substanz (X) 2 bis 20 Gew.-% beträgt, und zwar bezogen auf die Gesamtmasse des Adduktes (3).

13. Werkstoff nach einem der Ansprüche 1 bis 12, **dadurch gekennzeichnet, dass** die Beladungsmenge des Antithrombotikums (Y) 1 bis 10 Gew.-% beträgt, und zwar bezogen auf die Gesamtmasse des Adduktes (3).

14. Werkstoff nach einem der Ansprüche 1 bis 13, **dadurch gekennzeichnet, dass** die Matrix (2) ein Polymerwerkstoff, vorzugsweise ein Elastomer, ein thermoplastisches Elastomer oder ein thermoplastischer Kunststoff ist.

15. Werkstoff nach einem der Ansprüche 1 bis 14, **dadurch gekennzeichnet, dass** das Molekularsieb mit weiteren Stoffen, vorzugsweise Mineralien, beladen ist.

16. Verfahren zur Herstellung eines Werkstoffes nach einem der Ansprüche 1 bis 15, **gekennzeichnet durch** folgende Verfahrensschritte:
- das Molekularsieb mit einer Grundmolmenge (m) an Kristallwasser von wenigstens 25 wird bei 200 bis 300°C, vorzugsweise bei etwa 250°C, mehrere Stunden, vorzugsweise 2 bis 6 Stunden, lang bei einem Dehydratisierungsgrad von mindestens 20% partiell dehydratisiert;
- anschließend wird das partiell dehydratisierte Molekularsieb mit der reduzierten Molmenge (m') an Kristallwasser mit wenigstens einer körpereigenen Substanz (X) und dem Antithrombotikum (Y) sowie gegebenenfalls mit weiteren Stoffen unter Bildung des Adduktes (3) beladen;
- schließlich wird das Addukt (3) in die Matrix (2) eingearbeitet bzw. matrixmäßig verarbeitet.

17. Verfahren zur Herstellung eines Werkstoffes nach Anspruch 16, **dadurch gekennzeichnet, dass** die Dehydratisierung und/oder Beladung in Gegenwart eines inerten Gases, beispielsweise von Stickstoff, durchgeführt wird/werden.

18. Verfahren zur Herstellung eines Werkstoffes nach Anspruch 16 oder 17, **dadurch gekennzeichnet, dass** die Dehydratisierung und/oder Beladung unter Normaldruck durchgeführt wird/werden.

## Claims

1. Material for limited selective adsorption and/or absorption of endogenous substances in the blood which are metabolically harmful when the corresponding standard values are exceeded, comprising at least:
- a blood-permeable and blood-compatible matrix (2), in particular in the form of a membrane (1), and
- a carrier in the form of a molecular sieve which is incorporated in the matrix (2) or processed in matrix form and charged with at least one endogenous substance (X) and also with an anti-thrombotic (Y), forming a molecular sieve/substance/anti-thrombotic adduct (3), in which the concentration of the charging endogenous substance (X) lies below its standard value so that on the basis of the drop in concentration of this substance within the system of blood (4) and matrix (2) the metabolically harmful substance (X') is adsorbed and/or absorbed until the standard value is restored, and in which further the anti-thrombotic (Y) is also released desorptively at the same time,
**characterised in that**
- the molecular sieve/substance/anti-thrombotic adduct (3) additionally contains water of crystallisation, such that the molecular sieve is partially dehydrated with a degree of dehydration of at least 20°s in relation to a basic molar quantity (m) of water of crystallisation of at least 25, the molecular sieve with the reduced molar quantity (m') of water of crystallisation being charged with the endogenous substance (X) and the anti-thrombotic (Y) so that when the matrix (2) comes into contact with the blood, adsorption of water and desorption of the anti-thrombotic take place, and the content of water of crystallisation of the molecular sieve increases.

2. Material according to claim 1, **characterised in that** the molecular sieve is a metal-aluminium silicate with the following formula:
Meₙ[(AlO₂) . (SiO₂)_{Y}] . m'H₂O

3. Material according to claim 2, **characterised in that** a metal of the first or second main group of the periodic system, preferably sodium, is used.

4. Material according to claim 2 or 3, **characterised in that** the molecular sieve is a sodium-aluminium silicate with the following formula:
Na₁₂[(AlO₂)₁₂ . (SiO₂)₁₂] . m'H₂O

5. Material according to claim 2 or 3, **characterised in that** the molecular sieve is a sodium-aluminium silicate with the following formula:
Na₈₆[(AlO₂)₈₆ . (SiO₂)₁₀₆] . m'H₂O

6. Material according to one of claims 1 to 5, **characterised in that** the molecular sieve contains a basic molar quantity (m) of water of crystallisation of at least 100, in particular at least 200.

7. Material according to one of claims 1 to 6, **characterised in that** the partially dehydrated molecular sieve exhibits a degree of dehydration of 30% to 70%.

8. Material according to one of claims 1 to 7, **characterised in that** the total degree of charging of the endogenous substance (X) and the anti-thrombotic (Y) is less than the degree of dehydration of the partially dehydrated molecular sieve.

9. Material according to claim 8, **characterised in that** the total degree of charging is at least 20% of the degree of dehydration.

10. Material according to one of claims 1 to 9, **characterised in that** the adduct (3) is essentially uniformly distributed inside the matrix (2).

11. Material according to one of claims 1 to 10, **characterised in that** the adduct (3) exhibits a level of 2 to 30% by weight, preferably 15 to 25% by weight, in relation to the total mass of the matrix (2).

12. Material according to one of claims 1 to 11, **characterised in that** the quantity of the charge of the endogenous substance (X) is 2 to 20% by weight, in relation to the total mass of the adduct (3).

13. Material according to one of claims 1 to 12, **characterised in that** the quantity of the charge of the anti-thrombotic (Y) is 1 to 10% by weight, in relation to the total mass of the adduct (3).

14. Material according to one of claims 1 to 13, **characterised in that** the matrix (2) is a polymer material, preferably an elastomer, a thermoplastic elastomer or a thermoplastic plastic.

15. Material according to one of claims 1 to 14, **characterised in that** the molecular sieve is charged with further substances, preferably minerals.

16. Method for manufacturing a material according to one of claims 1 to 15, **characterised by** the following process steps:
- the molecular sieve with a basic molar quantity (m) of water of crystallisation of at least 25 is partially dehydrated at 200 to 300°C, preferably at approximately 250°C, for a plurality of hours, preferably 2 to 6 hours, with a degree of dehydration of at least 20%;
- then the partially dehydrated molecular sieve with the reduced molar quantity (m') of water of crystallisation is charged with at least one endgogenous substance (X) and the anti-thrombotic (Y) and possibly with further substances to form the adduct (3);
- finally, the adduct (3) is incorporated in the matrix (2) or processed in matrix form.

17. Method for manufacturing a material according to claim 16, **characterised in that** the dehydration and/or charging is carried out in the presence of an inert gas, for example nitrogen.

18. Method for manufacturing a material according to claim 16 or 17, **characterised in that** the dehydration and/or charging is carried out at normal pressure.

## Revendications

1. Matériau pour l'adsorption et/ou l'absorption sélective limitée de substances endogènes dans le sang, qui dans le cas d'un dépassement des valeurs normalisées correspondantes ont un effet nocif sur le métabolisme, comprenant au moins :
- une matrice (2), perméable au sang et compatible avec le sang, en particulier sous forme d'une membrane (1) ; et aussi
- un support, sous forme d'un tamis moléculaire, qui est incorporé dans la matrice (2) ou a été mis en oeuvre sous forme d'une matrice, et qui est chargé d'au moins une substance endogène (X) et en outre d'un antithrombotique (Y), et plus précisément avec formation d'un adduit (3) tamis moléculaire/substance/antithrombotique, la concentration de la substance endogène chargée (X) étant inférieure à sa valeur normalisée, de sorte que, sur la base du gradient de concentration de cette substance à l'intérieur du système du sang (4) et de la matrice (2), il y ait adsorption et/ou absorption de la substance (X') nocive pour le métabolisme, jusqu'à rétablissement de la valeur normalisée, l'antithrombotique (Y) subissant en outre et simultanément une libération par désorption,
**caractérisé en ce que**
- l'adduit (3) tamis moléculaire/substance/antithrombotique contient en outre de l'eau de cristallisation, et plus précisément de telle sorte que, par rapport à une quantité molaire de base (m) d'eau de cristallisation d'au moins 25, le tamis moléculaire est partiellement déshydraté pour un degré de déshydratation d'au moins 20 %, le tamis moléculaire étant chargé de la quantité molaire réduite (m') d'eau de cristallisation avec la substance endogène (X) et l'antithrombotique (Y) de telle sorte que, après contact de la matrice (2) avec le sang, il se produise une adsorption de l'eau avec désorption de l'antithrombotique, la teneur du tamis moléculaire en eau de cristallisation subissant une augmentation.

2. Matériau selon la revendication 1, **caractérisé en ce que** le tamis moléculaire est un silicate de métal et d'aluminium ayant la formule suivante :
Meₙ[(AlO₂)ₓ . (SiO₂)_{y}] . m'H₂O

3. Matériau selon la revendication 2, **caractérisé en ce qu'**il contient un métal du premier ou du deuxième groupe principal du Tableau Périodique, de préférence le sodium.

4. Matériau selon la revendication 2 ou 3, **caractérisé en ce que** le tamis moléculaire est un silicate de sodium et d'aluminium ayant la formule suivante :
Na₁₂[(AlO₂)₁₂ . (SiO₂)₁₂] . m'H₂O

5. Matériau selon la revendication 2 ou 3, **caractérisé en ce que** le tamis moléculaire est un silicate de sodium et d'aluminium ayant la formule suivante :
Na₈₆[(AlO₂)₈₆ . (SiO₂)₁₀₆] . m'H₂O

6. Matériau selon l'une des revendications 1 à 5, **caractérisé en ce que** le tamis moléculaire contient une quantité molaire de base (m) d'eau de cristallisation d'au moins 100 et en particulier d'au moins 200.

7. Matériau selon l'une des revendications 1 à 6, **caractérisé en ce que** le tamis moléculaire partiellement déshydraté présente un degré de déshydratation de 30 à 70 %.

8. Matériau selon l'une des revendications 1 à 7, **caractérisé en ce que** le degré total de charge de la substance endogène (X) et de l'antithrombotique (Y) est inférieur au degré de déshydratation du tamis moléculaire partiellement déshydraté.

9. Matériau selon la revendication 8, **caractérisé en ce que** le degré total de charge est d'au moins 20 % du degré de déshydratation.

10. Matériau selon l'une des revendications 1 à 9, **caractérisé en ce que** l'adduit (3) est réparti d'une manière essentiellement uniforme à l'intérieur de la matrice (2).

11. Matériau selon l'une des revendications 1 à 10, **caractérisé en ce que** l'adduit (3) est présent en une quantité de 2 à 30 % en poids et de préférence de 15 à 25 % en poids par rapport à la masse totale de la matrice (2).

12. Matériau selon l'une des revendications 1 à 11, **caractérisé en ce que** la quantité de charge de la substance endogène (X) est de 2 à 20 % en poids par rapport à la masse totale de l'adduit (3).

13. Matériau selon l'une des revendications 1 à 12, **caractérisé en ce que** la quantité de charge de l'antithrombotique (Y) est de 1 à 10 % en poids par rapport à la masse totale de l'adduit (3).

14. Matériau selon l'une des revendications 1 à 13, **caractérisé en ce que** la matrice (2) est un matériau polymère, de préférence un élastomère, un élastomère thermoplastique ou un matériau plastique thermoplastique.

15. Matériau selon l'une des revendications 1 à 14, **caractérisé en ce que** le tamis moléculaire est chargé d'autres substances, de préférence des substances minérales.

16. Procédé de préparation d'un matériau selon l'une des revendications 1 à 15, **caractérisé par** les étapes suivantes :
- le tamis moléculaire, avec une quantité molaire de base (m) d'eau de cristallisation d'au moins 25, est partiellement déshydraté à une température de 200 à 300°C et de préférence d'environ 250°C pendant plusieurs heures et de préférence pendant 2 à 6 heures pour un degré de déshydratation d'au moins 20 % ;
- puis le tamis moléculaire partiellement déshydraté, contenant la quantité molaire réduite (m') d'eau de cristallisation, est chargé au moins une substance endogène (X) et de l'antithrombotique (Y), et éventuellement d'autres substances, pour former l'adduit (3) ;
- finalement, l'adduit (3) est incorporé dans la matrice (2) ou est mis en oeuvre à la manière d'une matrice.

17. Procédé de préparation d'un matériau selon la revendication 16, **caractérisé en ce que** la déshydratation et/ou la charge sont mises en oeuvre en présence d'un gaz inerte, par exemple l'azote.

18. Procédé de préparation d'un matériau selon la revendication 16 ou 17, **caractérisé en ce que** la déshydratation et/ou la charge sont mises en oeuvre sous la pression normale.
